# EUROPÄISCHE PATENTSCHRIFT

(11) **EP 2 200 974 B1**
(45) Veröffentlichungstag und Bekanntmachung des Hinweises auf die Patenterteilung: **29.01.2014**
(21) Anmeldenummer: 08785575.5
(22) Anmeldetag: 15.08.2008
(51) Int. Cl.: C07C 253/04, C07C 253/34, C07C 255/05

(54) **VERFAHREN ZUR HERSTELLUNG UND REINIGUNG VON ALKALI- UND ERDALKALIMETALLTRICYANOMETHANIDEN**
PROCESS FOR PREPARING AND PURIFYING ALKALI METAL AND ALKALINE EARTH METAL TRICYANOMETHANIDES
PROCÉDÉ DE FABRICATION ET DE PURIFICATION DE TRICYANOMÉTHANURES DE MÉTAUX ALCALINS ET ALCALINO-TERREUX

(30) Priorität: 16.08.2007 EP 07016100; 26.10.2007 EP 07020980
(43) Veröffentlichungstag der Anmeldung: 30.06.2010
(73) Patentinhaber: Lonza Ltd, 4052 Basel (CH)
(72) Erfinder: STRITTMATTER, Harald, CH-3930 Visp (CH); KOGER, Stefan, CH-3930 Visp (CH)
(86) Internationale Anmeldenummer: PCT/EP2008/006730
(87) Internationale Veröffentlichungsnummer: WO 2009/021751

(56) Entgegenhaltungen:
- WO-A-2006/021390
- COX E ET AL: "LE CYANOFORME OU TRICYANOMETHANE. NOUVELLE METHODE DE PREPARATION" BULLETIN DE LA SOCIETE CHIMIQUE DE FRANCE, SOCIETE FRANCAISE DE CHIMIE. PARIS, 1. Januar 1954 (1954-01-01), Seiten 948-950, XP009085459 ISSN: 0037-8968 in der Anmeldung erwähnt
- SCHMIDTMANN H: "UEBER EINIGE DERIVATE DES MALONITRILS" CHEMISCHE BERICHTE, VERLAG CHEMIE GMBH. WEINHEIM, DE, Bd. 29, 1. Januar 1896 (1896-01-01), Seiten 1168-1175, XP009044882 ISSN: 0009-2940 in der Anmeldung erwähnt
- HIPPS K W ET AL: "The tricyanomethanide ion: an infrared, Raman, and tunneling spectroscopy study including isotopic substitution" JOURNAL OF PHYSICAL CHEMISTRY, AMERICAN CHEMICAL SOCIETY, US, Bd. 89, Nr. 25, 1. Januar 1985 (1985-01-01), Seiten 5459-5464, XP002472119 ISSN: 0022-3654 in der Anmeldung erwähnt

## Beschreibung

Die vorliegende Erfindung betrifft ein Verfahren zur Herstellung von Alkali- und Erdalkalimetalltricyanomethaniden mit einer besonders hohen Reinheit.

Ionische Flüssigkeiten, enthaltend Alkali- oder Erdalkalimetalltricyanomethanide (TCM), sind für die elektronischen Industrie wichtige Roh- und Hilfsstoffe u.a. für die Herstellung von wiederaufladbaren Batterien. Für die meisten Anwendungsgebiete ist es dabei erforderlich, dass die verwendeten Methanide besonders rein, insbesondere halogenidfrei, sind, um Korrosionsprobleme und/oder ungewollte Nebenreaktionen zu vermeiden.

Es sind verschiedene Verfahren zur Herstellung von Tricyanomethaniden bekannt. Die Cyanidierung von Malonodinitril (MDN) wurde erstmal von Schmidtmann in Chem. Ber. 1896, 29, 1168-1175 beschrieben. Hierbei wird MDN mit Natriumethanolat in Ethanol deprotoniert und durch schrittweise Zugabe von Chlorcyan zu Natriumtricyanomethanid umgesetzt und anschliessend aus Ether kristallisiert. Bei dem Verfahren wird Natriumtricyanomethanid in einer Ausbeute von ca. 70 % isoliert.

Birckenbach et al. offenbarten in Chem. Ber. 1929, 62B, 153-163 die Cyanidierung von MDN mit Bromcyan. Birckenbach et al. und Mayer et al. (Monatsh. Chem., 1969, 100, 462) haben die Herstellung von halogenarmem Silbertricyanomethanid beschrieben, indem rohes Alkalimetalltricyanomethanid mit Silbernitrat versetzt wurde, wobei zuerst Silberchlorid oder Silberbromid ausfiel. Aus dem Filtrat konnte nach weiterer Silbernitratzugabe Silbertricyanomethanid isoliert werden. Mayer et al. haben im Weiteren die Umsetzung von Silbertricyanomethanid mit Chlorcyan über 40 Stunden bei 100 °C zu Tetracyanomethan beschrieben, welches sublimiert und anschliessend in Schwefelsäure zu Ammoniumtricyanomethanid hydrolysiert wurde. Lithiumtricyanomethanid wurde von Mayer durch Zugabe von Lithiumchlorid zu einer Acetonitrillösung von Tetracyanomethan bei -96°C erhalten.

Die Herstellung hochreinen Kaliumtricyanomethanids wurde erstmals von Hipps et al. offenbart (J. Phys. Chem. 1985, 89, 5459). In dem Verfahren wurde Kaliumtricyanomethanid in Aceton gelöst, die Lösung mit Aktivkohle behandelt und anschliessend das Kaliumtricyanomethanid in Diethylether ausgefällt. Diese Prozedur wurde 10-mal wiederholt. Um Reste von organischen Verunreinigungen zu entfernen, wurde das erhaltene Kaliumtricyanomethanid anschliessend noch zweimal aus Wasser umkristallisiert. Hierbei wurde ein weisses kristallines Pulver erhalten, das bei einer Anregung mit Licht von 5145Å keinen Ramanfluoreszenz-Hintergrund zeigte und als hochrein interpretiert wurde.

In WO-A-98/29389 wurde die Cyanidierung von MDN mit Bromcyan in Gegenwart von 1,4-Diazabicyclo[2.2.2]octan (DABCO^{®}) in THF offenbart. Bei dem Verfahren wird DABCO^{®}-Hydrochlorid innerhalb von 28 h bei -20°C auskristallisiert. Hierbei wurde 98%-iges Lithiumtricyanomethanid erhalten.

Ein weiteres Verfahren wurde von Trofimenko et al. in J. Org. Chem. 1962, 27, 433 offenbart, worin Kaliumtricyanomethanid durch Behandlung eines Dihalogenmalonodinitril-Kaliumbromid-Komplexes mit Kaliumcyanid erhalten wurde.

Cox et al. haben in Bull. Soc. Chim. Fr. 1954, 948 ein weiteres Verfahren zur Herstellung von Tricyanomethan bei tiefer Temperatur beschrieben, wobei Brommalonodinitril mit Kaliumcyanid umgesetzt wurde.

Weitere Verfahren zur Herstellung von Tricyanomethaniden, enthaltend die Umsetzung von deprotoniertem MDN mit Phenylcyanat wurden von Grigat et al. in Chem. Ber. 1965, 98, 3777-3784 und Martin et al. in DD-A-48614 offenbart. Hierbei wurden Ausbeuten von 75 bis 88% erhalten.

Ein weiteres Verfahren zur Reinigung von Natriumtricyanomethanid durch Umkristallisation in Acetonitril wurde 1987 von Bock et al. in Z. Naturforsch., 1987, 42b, 315 beschrieben, wobei Natriumtricyanomethanid in 70%-iger Ausbeute erhalten wurde (ohne Angabe der Reinheit).

Keines der Verfahren liefert halogenfreie Produkte. Die erhältlichen Tricyanomethanide müssen immer in mehr oder weniger aufwändigen Reinigungsschritten nachbearbeitet werden. Der Erfolg dieser Aufreinigung hängt unter anderem vom Verunreinigungsprofil, dem Produktrohgehalt und der Konsistenz des Produktes ab.

Aufgabe der vorliegenden Erfindung war die Bereitstellung eines einfachen Verfahrens zur Herstellung von weitgehend nebenproduktfreien Tricyanomethaniden. Das Verfahren sollte sich für die grosstechnische Produktion eignen. Hierfür sollte das Rohprodukt bereits einen hohen Gehalt an Tricyanomethanide enthalten und möglichst kristallin anfallen.

Die Aufgabe wurde entsprechend Anspruch 1 gelöst.

Beansprucht wird ein Verfahren zur Herstellung von Alkali- und Erdalkalimetalltricyanomethaniden in einer Reinheit von mindestens 99 Gew.-%, wobei in Gegenwart einer Alkali- oder Erdalkalimetallbase deprotoniertes Malonodinitril in einem wässrigen Lösungsmittelgemisch aus mindestens einem organischen Lösungsmittel und Wasser bei höchstens 35 °C, vorteilhaft bei nicht mehr als 30 °C, besonders vorteilhaft bei nicht mehr als 20 °C, mit einem Halogencyan umgesetzt und ausgefallenes Alkali- oder Erdalkalimetallhalogenid, vorzugsweise nach vollständiger Zugabe des Halogencyans, abgetrennt wird und worin in einem weiteren Schritt das wässrige Lösungsmittelgemisch wenigstens soweit abdestilliert wird, bis das Alkali- oder Erdalkalimetalltricyanomethanid ausfällt. Vorzugsweise fällt das Alkali- oder Erdalkalimetalltricyanomethanid im erfindungsgemässen Verfahren in Form grobkörniger Partikel aus.

In zwei Verfahrensvarianten ist es einerseits möglich, dass vor der Zugabe des Halogencyans das Malonodinitril zusammen mit der Alkali- oder Erdalkalimetallbase vorgelegt und deprotoniert wird oder andererseits, dass das Malonodinitril vordosiert und das Halogencyan zeitverzögert zudosiert wird, wobei die Zudosierung von Malonodinitril und Halogencyan über eine bestimmte Zeit parallel erfolgt.

Das gemäss beiden Verfahrensvarianten erhaltene Produkt enthält in jedem Fall weniger Nebenprodukte, als bei einer entsprechenden Reaktion in Wasser. Gegenüber einer Reaktion in Wasser hat die Reaktionsführung in einem Lösungsmittelgemisch aus mindestens einem organischen Lösungsmittel und Wasser den Vorteil, dass der pH der Reaktionsmischung nicht in einem konstanten und engen Bereich gehalten werden muss. Es muss während der Reaktion nur eine ausreichende Deprotonierung des MDN gewährleistet sein. Die hierfür benötigte Menge Base lässt sich in bekannter Weise aus dem pKₐ der verwendeten Verbindungen ermitteln.

Überraschenderweise wurde gefunden, dass Tricyanomethanide in hoher Ausbeute als Feststoffe erhalten werden können, wenn sichergestellt ist, dass während der Reaktion das Malonodinitril (MDN) in einem basischen Milieu vorliegt, in dem MDN weitgehend vollständig deprotoniert ist. Dies kann durch Vorlegen des MDN in einer basischen Lösung und Zudosierung eines Halogencyans oder durch Paralleldosierung von MDN und dem Halogencyan in ein basisches Reaktionsgemisch erreicht werden. Insbesondere bei der Paralleldosierung ist dabei zu beachten, dass MDN nur so langsam zudosiert werden sollte, wie das deprotonierte MDN mit dem Halogencyan reagiert. Da die Umsetzung sehr exotherm ist, muss für eine gute Wärmeabfuhr Sorge getragen werden. Bei der erfindungsgemässen Umsetzung wird NaTCM mit besonders wenig Nebenprodukten erhalten. In einer vorteilhaften Verfahrensvariante wird etwas MDN vordosiert und das Halogencyan zeitverzögert zudosiert.

Das Verfahren mit vorgelegtem MDN ist vorteilhaft gegenüber dem der Paralleldosierung, weil das parallele Dosieren technisch aufwändiger ist. Da wie oben erwähnt die Zugabe von Chlorcyan zu vollständig deprotoniertem MDN in Acetonitril stark exotherm verläuft und eine starke und zuverlässige Kühlung der Reaktionsmischung voraussetzt, ist das Verfahren mit einer Paralleldosierung einfacher bezüglich der Temperaturkontrolle.

Als Halogencyan kann Chlor-, Bromcyan verwendet werden. Vorzugsweise wird Chlorcyan als Halogencyan verwendet. Besonders bevorzugt wird das Halogencyan im Verhältnis zu Malonodinitril von 1:1 bis 10:1, vorzugsweise von 1:1: bis 1:3 verwendet. Ein leichter Überschuss an Halogencyan ist bevorzugt.

Weiterhin ist die Alkali- oder Erdalkalimetallbase besonders bevorzugt eine starke Base. Insbesondere können hierbei Alkali- oder Erdalkalimetallhydroxide, Alkali- oder Erdalkalimetalloxide, Alkali- oder Erdalkalimetallalkoxide verwendet werden. Als Alkali- oder Erdalkalimetallalkoxide können insbesondere C₁₋₆-Alkoxide verwendet werden. Geeignete Alkali- oder Erdalkalimetall-C₁₋₆-Alkoxide sind beispielsweise Natrium oder Kaliumsalze der C₁₋₆-Akohole wie Methanol, Ethanol, Isopropanol, *n*-Butanol, *sec*-Butanol, *tert*-Butanol, Pentanol und Hexanol.

In einer bevorzugten Verfahrensvariante ist die Alkali- oder Erdalkalimetallbase eine Lithium-, Natrium- Kalium-, Calcium-, Magnesium- oder Bariumbase.

Besonders bevorzugt ist die Alkali- oder Erdalkalimetallbase ein Alkali- oder Erdalkalimetallhydroxid, Alkali- oder Erdalkalimetalloxid, ein Alkali- oder Erdalkalimetallalkoxid, oder eine Mischung daraus.

In einer besonders bevorzugten Ausführungsvariante weist das Lösungsmittelgemisch aus mindestens einem organischen Lösungsmittel und Wasser einen Siedepunkt bei 1 bar von höchstens 95 °C. Der Siedepunkt des in den Beispielen verwendeten Azeotrops Acetonitril-Wasser liegt bei 76 °C.

Weiterhin wird ein Verfahren zur Umkristallisation von Alkali- oder Erdalkalimetalltricyanomethaniden beansprucht, worin ein Alkali- oder Erdalkalimetalltricyanomethanid in einem wässrigen Lösungsmittelgemisch mit einem Siedepunkt bei 1 bar von höchstens 95 °C, vorgelegt, gegebenenfalls ausfallendes Alkali- oder Erdalkalimetallhalogenid abfiltriert und das Lösungsmittel wenigstens soweit abdestilliert wird, bis das Produkt ausfällt.

Vorzugsweise enthält das wässrige Lösungsmittelgemisch bei der Reaktion und/oder der Kristallisation als wesentlichen organischen Bestandteil mindestens einen Ether oder Alkohol, oder ein Keton, Formamid und/oder ein organisches Nitril.

Besonders bevorzugte Lösungsmittel sind beispielsweise 2-Propanol, sec-Butanol, Pentanol, Ethylenglykol, *tert*-Butanol, Aceton, Cyclopentanon, Methylethylketon, Methylisobutylketon, Methyl-*tert*-butylether, Diethylether, Diisopropylether, THF, Methyltetrahydrofuran, Dioxan, Diglyme, Ethylenglykoldiethylether und Ethylenglykoldiethylether, Dimethylformamid, Acetonitril, Butyronitril, Propionitril, Valeronitril und Mischungen daraus. In einer bevorzugten Verfahrensausführung enthält das wässrige Lösungsmittelgemisch ein Nitril, besonders bevorzugt Acetonitril. Bewährt hat sich beispielsweise ein Acetonitril/WasserGemisch mit einem Azeotropsiedepunkt bei 1 bar von 76 °C.

In einer bevorzugten Verfahrensvariante wird im zweiten Schritt das Alkali- oder Erdalkalimetalltricyanomethanid zunächst, gegebenenfalls bei erhöhter Temperatur, in Aceton, Methylethylketon und/oder Methylisobutylketon gelöst und, gegebenenfalls bei erniedrigter Temperatur, ausgefällt. Hierbei hat es sich als vorteilhaft herausgestellt, wenn die Alkalimetalltricyanomethanidlösung ausserdem Aktivkohle enthält. Die Aktivkohle wird dann abfiltriert, bevor das Alkali- oder Erdalkalimetalltricyanomethanid ausgefällt wird. In einer weiteren bevorzugten Verfahrensvariante wird das Alkali- oder Erdalkalimetalltricyanomethanid, gegebenenfalls nach Abtrennung der Aktivkohle, in Gegenwart von Methyl*tert*-butylether oder Diisopropylether, ausgefällt. Vorzugsweise wird das Alkali- oder Erdalkalimetalltricyanomethanid bei einer Temperatur unterhalb 20 °C, besonders bevorzugt bei 10 °C oder weniger ausgefällt.

In einem bevorzugten Verfahren wird das Alkali- oder Erdalkalimetalltricyanomethanid im zweiten Schritt in einer Reinheit von mindestens 99,5% erhalten, besonders bevorzugt in einer Reinheit von mindestens 99,8%. Ganz besonders bevorzugt können Alkali- oder Erdalkalimetalltricyanomethanide mit einer Reinheit von 99,9% und mehr erhalten werden.

In den Beispielen konnte beispielsweise Natriumtricyanomethanid mit einem Halogenidgehalt von weniger als 10 ppm erhalten werden.

### Beispiele

Bei den genannten Beispielen kann durch Ersatz der Natronlauge durch eine andere Alkali- oder Erdalkalimetallbase das entsprechende Alkali- oder Erdalkalimetalltricyanomethanid gewonnen werden. Durch Verwendung von Gemischen von Basen unterschiedlicher Alkali- oder Erdalkalimetalle können auch gemischte Alkali- oder Erdalkalimetalltricyanomethanide erhalten werden.

### Beispiel 1:

Wasser (5,70 kg) und Natriumhydroxidlösung (50 Gew.-%, 17,30 kg, 216 mol) wurden in Acetonitril (30,30 kg) vorgelegt und das Gemisch auf -5 °C temperiert. Nach Anlegen von ca. 900 mbar wurden im Verlauf von 7 h Malonodinitril (85%-ig in Methanol, 8,20 kg, 106 mol) und Chlorcyan (6,51 kg, 106 mol) zudosiert. Durch das Verhältnis Natriumhydroxid:Malonodinitril >2:1 und die lange Dosierdauer wurde sichergestellt, dass jeweils zudosiertes Malonodinitril sofort deprotoniert wurde. Weiterhin wurde die Temperatur in der Reaktionsmischung zwischen 0 und 20 °C gehalten. Nach vollständiger Zugabe wurde der Gehalt an Natriumtricyanomethan (NaTCM) auf 17,2 Gew.-% bestimmt. Anschliessend wurde die Reaktionsmischung auf 18 °C gebracht und das ausgefallene NaCl abzentrifugiert. Nach Abdestillation von 33 kg des wässrigen Lösungsmittelgemisches der Mutterlauge (65 kg) fiel das NaTCM aus. Es wurden 5,53 kg Tricyanomethan (49 mol) erhalten. Das grobkörnige Produkt war ohne Umkristallisation farblos und enthielt weniger als 0,5 Gew.-% Nebenprodukte sowie 1200 ppm NaCl.

### Beispiel 2:

Natriumhydroxidlösung (50 Gew.-%, 165 g, 2,1 mol) wurde in Acetonitril (267 g) vorgelegt und das Gemisch auf 25 °C temperiert. Dann wurden zuerst Malonodinitril (85 Gew.-% in Methanol, 77,7 g, 1 mol) und anschliessend Chlorcyan (62,1 g, 1,01 mol) jeweils im Verlauf von 1h zugegeben. Die Temperatur des Reaktors wurde so geregelt, dass in der Reaktionsmischung 25 °C nicht überschritten wurden. Nach vollständiger Zugabe von Chlorcyan wurde ausgefallenes NaCl bei RT abzentrifugiert. Die Mutterlauge enthielt 19,5 Gew.-% Produkt und weniger als 0,1 Gew.-% NaCl. Ungefähr die Hälfte des Lösungsmittels der Mutterlauge wurde im Vakuum entfernt, wobei das grobkörnige Produkt ohne Umkristallisation farblos ausfiel und weniger als 0,5 Gew.-% Nebenprodukte sowie 1300 ppm NaCl enthielt.

### Beispiel 3:

Natriumhydroxidlösung (50 Gew.-%, 19,4 kg, 243 mol) wurde in Acetonitril (30,52 kg) und Wasser (5,74 kg) vorgelegt und das Gemisch auf 18 °C temperiert. Dann wurden zunächst eine Portion Malonodinitril (MDN, 85 Gew.-% in Methanol, 460 g, 5,9 mol) vordosiert, um einen Überschuss an deprotoniertem MDN zu erhalten. Anschliessend wurden MDN (85 Gew.-% in Methanol, 8,71 kg, 112.1 mol) über 365 min sowie Chlorcyan (100 Gew.-%, 7,54 kg, 123 mol) über 400 min unter Rühren zudosiert. Die Temperatur im Reaktor war während der Dosierzeit zwischen 13.5 bis 19.9 °C. Die Reaktionsmischung hatte während der Dosierzeit des MDN einen pH von 14. Kurz nach Ende der MDN-Dosierung begann der pH zu sinken. Bei pH 11,1 wurde die Chlorcyandosierung gestoppt. Die Kühlung wurde abgestellt und nach Anstieg der Temperatur auf 15 °C das während der Reaktion ausgefallene NaCl abzentrifugiert. Die Mutterlauge enthielt 18,7 Gew.-% Produkt (NaTCM) und 2,2 Gew.-% NaCl. Nach Abdestillation etwa der Hälfte des wässrigen Lösungsmittelgemisches der Mutterlauge (65 kg) fiel das NaTCM als grobkörnige farblose Festkörper aus. Das feuchte Rohprodukt enthielt ca. 70 Gew.-% NaTCM, entsprechend einer Ausbeute von ca. 44% NaTCM bezogen auf MDN.

### Beispiel 4:

Aus der Mutterlauge (65,29 kg) aus Beispiel 3 wurde bei einer Reaktortemperatur von ca. 20 bis 29 °C und einem Druck von ca. 65 bis 112 mbar ein Grossteil des Lösungsmittels (28,23 kg, ca. 34 L) azeotrop abdestilliert und der Rückstand anschliessend auf -5 °C gekühlt. Der Rückstand wurde zentrifugiert. Die Mutterlauge (ca. 27,4 kg) wies einen pH von ca. 10 auf. Der Filterkuchen wurde zweimal mit kaltem Acetonitril (3,5 kg) gewaschen. Nach 26 h Trocknung bei 50 mbar und 70 °C verblieben 6,0 kg Produkt in Form grobkörniger farbloser Kristalle mit einem Gehalt von 99,9 Gew.-% NaTCM. Der Chloridgehalt betrug vor der Umkristallisation ca. 1200 ppm.

### Beispiel 5:

Natriumhydroxidlösung (50 Gew.-%, 17,3 kg) wurde in Acetonitril (30,3 kg) und Wasser (5,7 kg) vorgelegt und das Gemisch auf 18 °C temperiert. Dann wurden Malonodinitril (MDN, 85 Gew.-% in Methanol, 8,2 kg, 105 mol) und gasförmiges Chlorcyan (ClCN, 100 Gew.-%, 6,51 kg, 106 mol) jeweils im Verlauf von 7 h zudosiert. Die Dosierung von ClCN wurde gegenüber der Dosierung von MDN um 15 min zeitverzögert gestartet, um einen Überschuss an deprotoniertem MDN zu erhalten. Die Temperatur im Reaktor lag während der Dosierzeit zwischen 12 und 19 °C. Die Reaktionsmischung hatte während der Dosierzeit des MDN einen pH zwischen 13 und 14. Kurz nach Ende der MDN-Dosierung begann der pH zu sinken. Bei pH 11,7 wurde die Chlorcyandosierung gestoppt. Das während der Reaktion ausgefallene NaCl wurde abzentrifugiert. Die Mutterlauge enthielt 17,2 Gew.-% Produkt (NaTCM) und 2,0 Gew.-% NaCl.

### Vergleichsbeispiel 1:

In einem Rührwerk wurden Malonodinitril (85%-ig in Methanol, 462 g, 5,95 mol), Wasser (2021 g) und Phosphorsäure (85%-ig, 57,1 g, 0,5 mol) gemischt. Anschliessend wurde mit Natronlauge (50%-ig) ein pH-Wert von 7,5 eingestellt. Bei 25 bis 30 °C wurde innerhalb von 2 h Chlorcyan (979 g, 15,92 mol) zudosiert, wobei der pH-Wert durch geregelte Zugabe von Natronlauge (50%-ig) auf 6,4 bis 7,5 gehalten wurde. Es bildete sich eine beige bis braune klare Lösung. Nach Zugabe der gesamten Menge an Chlorcyan wurde die Reaktionsmischung noch für weitere 30 min bei 25 bis 30 °C gerührt. Hierbei wurde der pH-Wert durch geregelte Zugabe von Natronlauge (50%-ig) auf 7,0 bis 7,5 gehalten. Anschliessend wurde mit Natronlauge ein pH von 8,5 eingestellt. Dann wurden 50 g Aktivkohle zur Reaktionsmischung gegeben. Die erhaltene Suspension wurde für weitere 30 min bei 25 bis 30 °C gerührt und anschliessend filtriert. Als Filtrat erhielt man 3950 g einer gelblichen Lösung bestehend mit 16,8 Gew.-% Natriumtricyanomethanid, 10,5 Gew.-% anorganischen Salzen, 71,5 Gew.-% Wasser, 1,3 Gew.-% Methanol und 0,1 Gew.-% organischer Verunreinigungen. Die Reinheit des Produktes betrug 98,6 Gew.-%.

### Vergleichsbeispiel 2:

Malonodinitril (169,6 g, 2,18 mol, 85%-ig in Methanol), Wasser (392,8 g) und Phosphorsäure (18,8 g, 0,16 mol 85%-ig) wurden gemischt. Anschliessend wurde mit Natronlauge (50%-ig) ein pH-Wert von 7,5 eingestellt. Bei 25 bis 30°C wurde innerhalb von 4 h Chlorcyan (137,8 g, 2,24 mol) zudosiert, wobei der pH-Wert durch geregelte Zugabe von Natronlauge (50%-ig) auf 7,3 bis 7,5 gehalten wurde. Nachdem die gesamte Menge an Chlorcyan zugegeben worden war, wurde die beige bis braune Reaktionsmischung noch für weitere 30 min bei 25 bis 30°C gerührt, wobei der pH Wert durch geregelte Zugabe von Natronlauge (50%-ig) auf 7,3 bis 7,5 gehalten wurde. Danach wurde der pH Wert mit Natronlauge auf 8,5 eingestellt und die Temperatur auf 70 °C erhöht. Dabei bildete sich aus der Suspension wieder eine klare beige bis braune Lösung. Diese Lösung wurde dann mit einer Rate von 6 °C/h auf 10 °C abgekühlt, wobei sich wieder eine Suspension bildete, die anschliessend zentrifugiert wurde.

### Beispiel 6:

Getrocknetes Produkt aus Vergleichsbeispiel 1 (65 g) wurden in Acetonitril (500 g) gelöst und anschliessend mit Wasser (100 g) versetzt. Bei einer Reaktortemperatur von ca. 20 bis 29 °C und einem Druck von ca. 65 bis 112 mbar wurde das Lösungsmittelgemisch azeotrop abdestilliert bis das Reaktionsgemisch eintrübte. Nach Zugabe von Methyl-tert-butylether wurde langsam weiter abdestilliert bis das Produkt anfing auszufallen und auf -5 °C gekühlt. Der Rückstand wurde zentrifugiert und der Filterkuchen zweimal mit kaltem Acetonitril gewaschen. Nach 26 h Trocknung bei 50 mbar und 70 °C wurde NaTCM in Form grobkörniger farbloser Kristalle erhalten mit einem Gehalt von 99,9 Gew.-% NaTCM.

### Beispiel 7:

In gleicher Weise wie in Beispiel 6 wurde aus Vergleichsbeispiel 2 NaTCM in Form grobkörniger farbloser Kristalle erhalten mit einem Gehalt von 99,9 Gew.-% NaTCM.

## Patentansprüche

1. Verfahren zur Herstellung von Alkali- und Erdalkalimetalltricyanomethaniden in einer Reinheit von mindestens 99 Gew.-%, wobei in Gegenwart einer Alkali- oder Erdalkalimetallbase deprotoniertes Malonodinitril in einem wässrigen Lösungsmittelgemisch aus mindestens einem organischen Lösungsmittel und Wasser bei höchstens 35 °C mit einem Halogencyan umgesetzt und ausgefallenes Alkali- oder Erdalkalimetallhalogenid abgetrennt wird und in einem weiteren Schritt das wässrige Lösungsmittelgemisch wenigstens soweit abdestilliert wird, bis das Alkali- oder Erdalkalimetalltricyanomethanid ausfällt.

2. Verfahren nach Anspruch 1, worin vor der Zugabe des Halogencyans das Malonodinitril zusammen mit der Alkali- oder Erdalkalimetallbase vorgelegt und deprotoniert wird.

3. Verfahren nach Anspruch 1, worin Malonodinitril vordosiert und das Halogencyan zeitverzögert zudosiert wird.

4. Verfahren nach einem der Ansprüche 1 bis 3, **dadurch gekennzeichnet, dass** das Halogencyan Chlorcyan ist.

5. Verfahren nach einem der Ansprüche 1 bis 4, **dadurch gekennzeichnet, dass** die Alkali- oder Erdalkalimetallbase ausgewählt ist aus einer Gruppe bestehend aus Alkali- oder Erdalkalimetallhydroxiden, Alkali- oder Erdalkalimetalloxiden und Alkali- oder Erdalkalimetallalkoxiden.

6. Verfahren nach einem der Ansprüche 1 bis 5, **dadurch gekennzeichnet, dass** die Base eine Alkali- oder Erdalkalimetallbase aus der Gruppe bestehend aus Lithium-, Natrium-, Kalium-, Calcium-, Magnesium- und Bariumbasen ist.

7. Verfahren nach einem der Ansprüche 1 bis 6, worin das Lösungsmittelgemisch einen Siedepunkt bei 1 bar von höchstens 95 °C hat.

8. Verfahren zur Umkristallisation von Alkali- oder Erdalkalimetalltricyanomethaniden, worin ein Alkali- oder Erdalkalimetalltricyanomethanid in einem wässrigen Lösungsmittelgemisch mit einem Siedepunkt bei 1 bar von höchstens 95 °C, vorgelegt, gegebenenfalls ausfallendes Alkali- oder Erdalkalimetallhalogenid abfiltriert und das Lösungsmittel wenigstens soweit abdestilliert wird, bis das Produkt ausfällt.

9. Verfahren nach einem der Ansprüche 1 bis 8, **dadurch gekennzeichnet, dass** das wässrige Lösungsmittelgemisch als wesentlichen organischen Bestandteil mindestens ein Lösungsmittel aus der Gruppe bestehend aus Ethern, Alkoholen, Ketonen, Formamiden und organischen Nitrilen enthält.

10. Verfahren nach Anspruch 9, **dadurch gekennzeichnet, dass** das wässrige Lösungsmittelgemisch mindestens ein organisches Lösungsmittel aus der Gruppe bestehend aus 2-Propanol, *sec*-Butanol, Pentanol, Ethylenglykol, *tert*-Butanol, Aceton, Cyclopentanon, Methylethylketon, Methylisobutylketon, Methyl-*tert*-butylether, Diethylether, Diisopropylether, THF, 2-Methyltetrahydrofuran, Dioxan, Diglyme, Ethylenglykoldiethylether und Ethylenglykoldiethylether, Dimethylformamid, Acetonitril, Propionitril, Butyronitril, Valeronitril und Mischungen daraus enthält.

## Claims

1. Process for preparing alkali metal and alkaline earth metal tricyanomethanides in a purity of at least 99% by weight, wherein malononitrile which has been deprotonated in the presence of an alkali metal base or alkaline earth metal base is reacted with a cyanogen halide in an aqueous solvent mixture comprising at least one organic solvent and water at not more than 35°C and precipitated alkali metal or alkaline earth metal halide is separated off and the aqueous solvent mixture is distilled off in a further step to at least such an extent that the alkali metal or alkaline earth metal tricyanomethanide precipitates.

2. Process according to Claim 1, wherein the malononitrile is initially charged together with the alkali metal or alkaline earth metal base and deprotonated before addition of the cyanogen halide.

3. Process according to Claim 1, wherein the introduction of malononitrile is commenced first and the introduction of the cyanogen halide is commenced after a time delay.

4. Process according to any of Claims 1 to 3, **characterized in that** the cyanogen halide is cyanogen chloride.

5. Process according to any of Claims 1 to 4, **characterized in that** the alkali metal or alkaline earth metal base is selected from the group consisting of alkali metal or alkaline earth metal hydroxides, alkali metal or alkaline earth metal oxides and alkali metal or alkaline earth metal alkoxides.

6. Process according to any of Claims 1 to 5, **characterized in that** the base is an alkali metal or alkaline earth metal base from the group consisting of lithium, sodium, potassium, calcium, magnesium and barium bases.

7. Process according to any of Claims 1 to 6, wherein the solvent mixture has a boiling point at 1 bar of not more than 95°C.

8. Process for recrystallizing alkali metal or alkaline earth metal tricyanomethanides, wherein an alkali metal or alkaline earth metal tricyanomethanide is initially charged in an aqueous solvent mixture having a boiling point at 1 bar of not more than 95°C, any alkali metal or alkaline earth metal halide which precipitates is filtered off and the solvent is distilled off to at least such an extent that the product precipitates.

9. Process according to any of Claims 1 to 8, **characterized in that** the aqueous solvent mixture contains as essential organic constituent at least one solvent from the group consisting of ethers, alcohols, ketones, formamides and organic nitriles.

10. Process according to Claim 9, **characterized in that** the aqueous solvent mixture contains at least one organic solvent from the group consisting of 2-propanol, sec-butanol, pentanol, ethylene glycol, *tert*-butanol, acetone, cyclopentanone, methyl ethyl ketone, methyl isobutyl ketone, methyl tert-butyl ether, diethyl ether, diisopropyl ether, THF, 2-methyltetrahydrofuran, dioxane, diglyme, ethyleneglycoldiethylether and ethyleneglycoldiethylether, dimethylformamide, acetonitrile, propionitrile, butyronitrile, valeronitrile and mixtures thereof.

## Revendications

1. Procédé pour la préparation de tricyanométhanures de métal alcalin et de métal alcalino-terreux en une pureté d'au moins 99% en poids, en transformant, en présence d'une base de métal alcalin ou de métal alcalino-terreux, du malonodinitrile déprotoné dans un mélange aqueux de solvants constitué par au moins un solvant organique et de l'eau, à au maximum 35°C, avec un halogénocyan et en séparant l'halogénure de métal alcalin ou de métal alcalino-terreux précipité et en éliminant par distillation dans une autre étape le mélange aqueux de solvants au moins jusqu'à ce que le tricyanométhanure de métal alcalin ou de métal alcalino-terreux précipite.

2. Procédé selon la revendication 1, dans lequel, avant l'addition de l'halogénocyan, on dispose au préalable le malonodinitrile ensemble avec la base de métal alcalin ou de métal alcalino-terreux et on déprotone.

3. Procédé selon la revendication 1, dans lequel le malonodinitrile est dosé au préalable et l'halogénocyan est dosé de manière retardée dans le temps.

4. Procédé selon l'une quelconque des revendications 1 à 3, **caractérisé en ce que** l'halogénocyan est le chlorocyan.

5. Procédé selon l'une quelconque des revendications 1 à 4, **caractérisé en ce que** la base de métal alcalin ou de métal alcalino-terreux est choisie dans un groupe constitué par les hydroxydes de métal alcalin ou de métal alcalino-terreux, les oxydes de métal alcalin ou de métal alcalino-terreux et les alcoxydes de métal alcalin ou de métal alcalino-terreux.

6. Procédé selon l'une quelconque des revendications 1 à 5, **caractérisé en ce que** la base est une base de métal alcalin ou de métal alcalino-terreux du groupe constitué par les bases de lithium, de sodium, de potassium, de calcium, de magnésium et de baryum.

7. Procédé selon l'une quelconque des revendications 1 à 6, dans lequel le mélange de solvants présente un point d'ébullition à 1 bar d'au plus 95°C.

8. Procédé pour la recristallisation de tricyanométhanures de métal alcalin ou de métal alcalino-terreux, dans lequel un tricyanométhanure de métal alcalin ou de métal alcalino-terreux est disposé au préalable dans un mélange aqueux de solvants présentant un point d'ébullition à 1 bar d'au plus 95°C, l'halogénure de métal alcalin ou de métal alcalino-terreux qui précipite le cas échéant est séparé par filtration et le solvant est éliminé par distillation au moins jusqu'à ce que le produit précipite.

9. Procédé selon l'une quelconque des revendications 1 à 8, **caractérisé en ce que** le mélange aqueux de solvants contient, comme constituant essentiellement organique, au moins un solvant du groupe constitué par les éthers, les alcools, les cétones, les formamides et les nitriles organiques.

10. Procédé selon la revendication 9, **caractérisé en ce que** le mélange aqueux de solvants contient au moins un solvant organique du groupe constitué par le 2-propanol, le sec-butanol, le pentanol, l'éthylèneglycol, le tert-butanol, l'acétone, la cyclopentanone, la méthyléthylcétone, la méthylisobutylcétone, le méthyl-tert-butyléther, le diéthyléther, le diisopropyléther, le THF, le 2-méthyltétrahydrofuranne, le dioxane, le diglyme, l'éthylèneglycoldiéthyléther et l'éthylèneglycoldiéthyléther, le diméthylformamide, l'acétonitrile, le propionitrile, le butyronitrile, le valéronitrile et leurs mélanges.
